(19) Europäisches Patentamt
European Patent Office
Office européen des brevets

(11) Veröffentlichungsnummer: **0 636 618 A1**

# (12) EUROPÄISCHE PATENTANMELDUNG

(21) Anmeldenummer: **94112856.3**

(22) Anmeldetag: **16.04.92**

(51) Int. Cl.[6]: **C07D 311/72**, A61K 31/355, A61K 9/107

Diese Anmeldung ist am 16 - 08 - 1994 als Teilanmeldung zu der unter INID-Kode 60 erwähnten Anmeldung eingereicht worden.

(30) Priorität: **04.06.91 CH 1662/91**

(43) Veröffentlichungstag der Anmeldung: **01.02.95 Patentblatt 95/05**

(60) Veröffentlichungsnummer der früheren Anmeldung nach Art. 76 EPÜ: **0 550 704**

(84) Benannte Vertragsstaaten: **DE GB**

(71) Anmelder: **MARIGEN S.A.**
**Eugster, Carl, Hackbergstrasse 40**
**CH-4125 Riehen (CH)**

(72) Erfinder: **Die Erfindernennung liegt noch nicht vor**

(74) Vertreter: **Haldemann, Walter, Dr.**
**c/o MARIGEN S.A.**
**40, Hackbergstrasse**
**CH-4125 Riehen (CH)**

## (54) Spontan dispergierbare Konzentrate und Mikroemulsionen mit Vitamin-E-Verbindungen zur Herstellung von Arzneimitteln mit Antitumoraler Wirksamkeit.

(57) Spontan dispergierbare Konzentrate mit Vitamin-E-Esterverbindungen, welche mit Wasser, physiologischer Kochsalz- oder Glucoselösung versetzt, Ultramikroemulsionen von hervorragender Phasenstabilität und von überaus deutlich erhöhtem Permeations- und Spreitungsvermögen ergeben; Verfahren zur Aufbereitung von geeigneten pharmazeutischen Darreichungsformen, sowie die Verwendung dieser Konzentrate und/oder Mikroemulsionen als Arzneimittel mit antitumoraler Wirksamkeit werden beschrieben.

EP 0 636 618 A1

Die vorliegende Erfindung betrifft spontan dispergierbare Konzentrate mit Vitamin-E-Verbindungen, welche mit Wasser, physiologischer Kochsalz- oder Glucoselösung versetzt, Ultramikroemulsionen von hervorragender Phasenstabilität und von überaus deutlich erhöhtem Permeations- und Spreitungsvermögen ergeben; Verfahren zur Aufbereitung von geeigneten pharmazeutischen Darreichungsformen, sowie ihre Verwendung als Arzneimittel mit antitumoraler Wirksamkeit.

*BESCHREIBUNG DER ERFINDUNG*

Die laut der vorliegenden Erfindung zu verwendenden Ester mit Vitamin-E-Verbindungen entsprechen den allgemeinen Formeln (I) und (II):

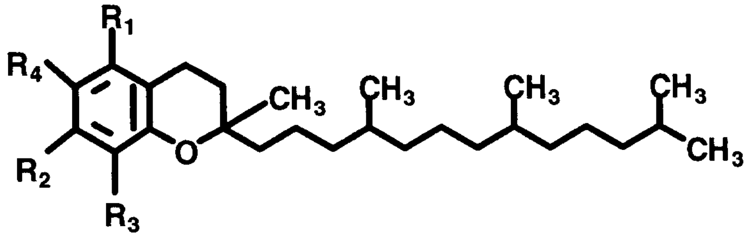

(I)

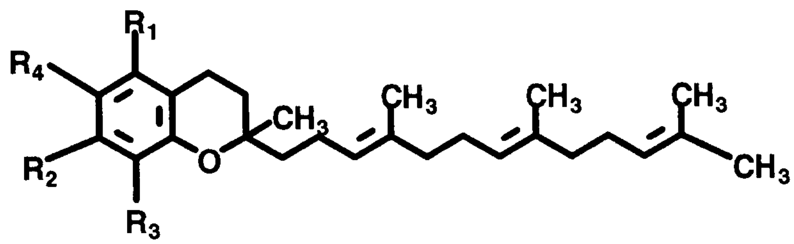

(II)

wobei die Radikale $R_1$, $R_2$ und $R_3$ Wasserstoff oder Methyl bedeuten und das Radikal $R_4$ eine $C_1$- bis $C_{32}$-Alkyl, bzw. eine $C_2$- bis $C_{32}$-Alkenyl- oder Alkapolyenkette mit funktioneller Carboxylgruppe oder eine Gruppe der Formeln (VII) oder (VIII) :

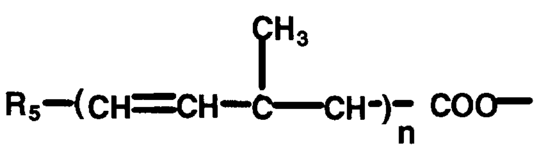

(VII)

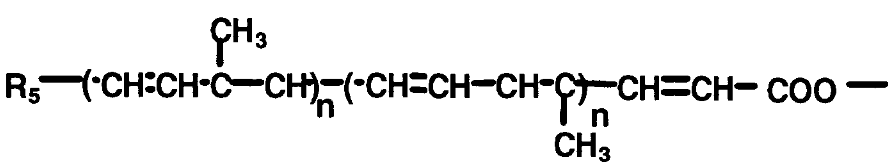

(VIII)

darstellt, worin n die Zahlen 1, 2, 3, 4 oder 5 bezeichnet und $R_5$ für ein Radikal der Formeln

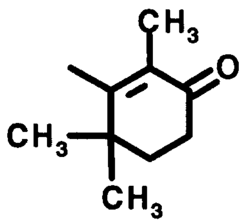
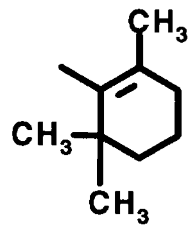
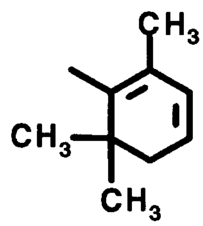

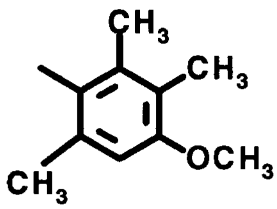
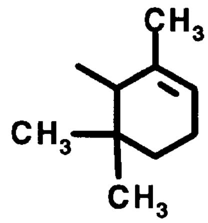
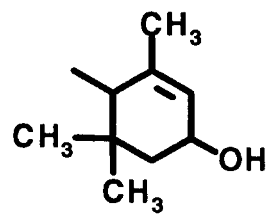

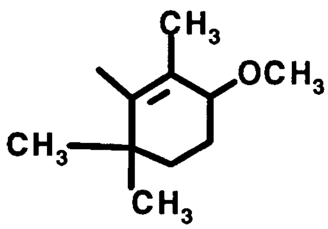
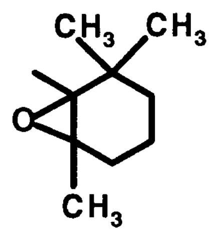
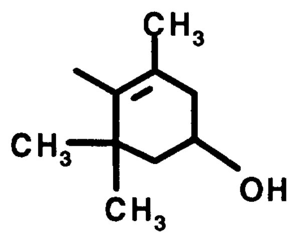

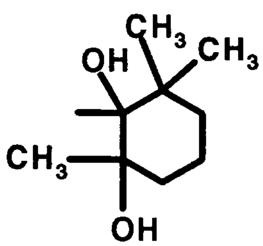
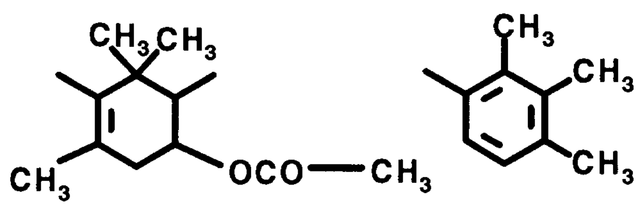

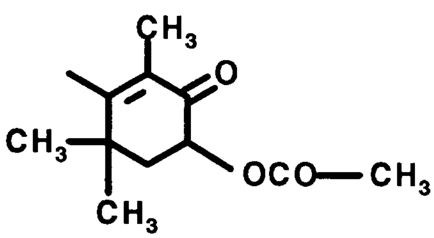
oder
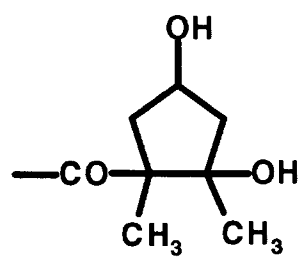

steht.

Die Gruppen der Formeln (I) und (II) können in verschiedenen stereoisomeren oder Drehformen vorliegen.

Unter Vitamin-E-Verbindungen sind alle Tocol- und Tocotrienolverbindungen wie z.B.:

Tocol [2-Methyl-2(4,8,12-Trimethyltridecyl)chroman-6-ol]

α-Tocopherol [5,7,8-Trimethyltocol]

welches In folgenden Konfigurationen vorliegen kann:

12,12,12-α-Tocopherol

2-epi-α-Tocopherol

2-ambo-α-Tocopherol

all-rac-α-Tocopherol

4-ambo-8-ambo-α-Tocopherol

β-Tocopherol [5,8-Dimethyltocol]

γ-Tocopherol [7,8-Dimethyltocol]

δ-Tocopherol [8-Methyltocol]

Tocotrienol [2-Methyl-2-(4,8,12-Trimethyldeca-3,7,11-trienyl) chroman-6-ol]

p1- oder p2-Tocopherol [5,7,8-Trimethyltocotrienol] und

ϵ-Tocopherol [5,8-Dimethyltocotrienol oder ϵ-Tocotrienol]

zu verstehen.

Die Alkylcarboxyl-, Alkenylcarboxyl- oder Alkapolyencarboxylgruppen bei $R^1$ können geradkettig oder verzweigt sein. Unter Alkapolyen sind die entsprechenden Alkadiene, Alkatriene, Alkatetraene, Alkapentaene, Alkahexaene und Alkaheptaene begriffen.

Wichtige Gruppen der Formel (V) sind gekennzeichnet durch die Formeln (VII), (VIII), bzw. (IX) :

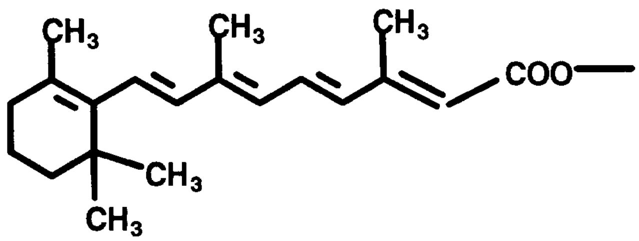

(VII)

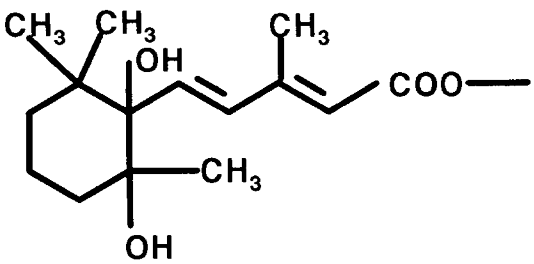

(VIII)

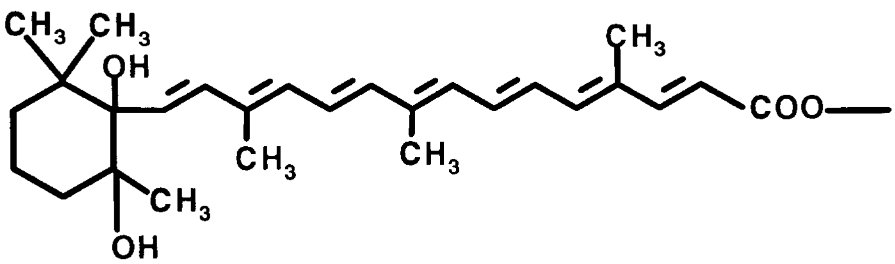

(IX)

Die Gruppe der Formel (VII) liegt in verschiedenen stereoisomeren Formen, so z.B. als all-trans, als 9-cis oder als 13-cis Form vor.

Beispiele von erfindungsgemäss zu verwendenden Estern mit Vitamin-E-Verbindungensind u.a.:

DL-α-Tocopheryl-Acetat
DL-α-Tocopheryl-Crotonat
DL-α-Tocopheryl-Caproylat
DL-α-Tocopheryl-nValerat
DL-α-Tocopheryl-Isovalerat
DL-α-Tocopheryl-Pelargonat
DL-α-Tocopheryl-10-Undecenoat
DL-α-Tocopheryl-Laurat
DL-α-Tocopheryl-2 Dodecenoat
DL-α-Tocopheryl-Myristat
DL-α-Tocopheryl-Palmitat
DL-α-Tocopheryl-Oleat
DL-α-Tocopheryl-Linolat
DL-α-Tocopheryl-Linolenat

DL-α-Tocopheryl-Arachidonat
DL-α-Tocopheryl-all trans-Retinat
DL-α-Tocopheryl-13-cis-Retinat

Die Ester mit Vitamin-E-Verbindungen der Formeln (I) und (II) lassen sich allgemein nach folgenden, an sich bekannten Verfahren herstellen:

a) Umsetzung einer Verbindung der Formeln (X) und (XI):

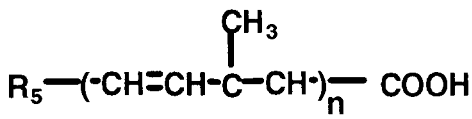

(X)

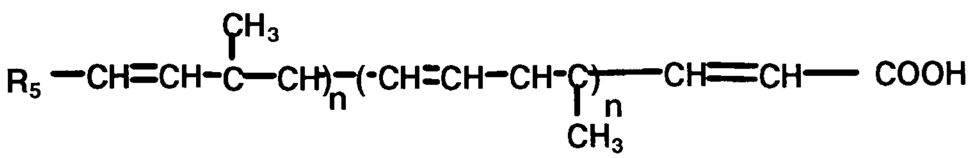

(XI)

worin n in den Zahlen 1, 2, 3, 4 oder 5 ist und $R_5$ für ein Radikal der Formeln:

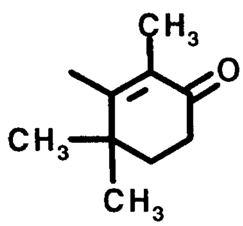
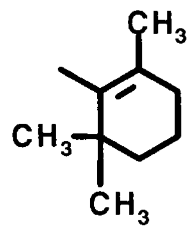
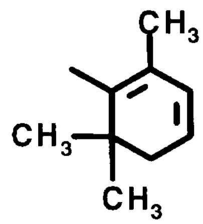
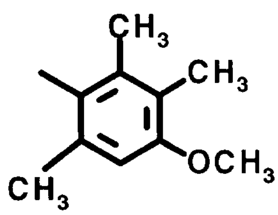
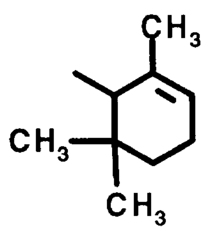
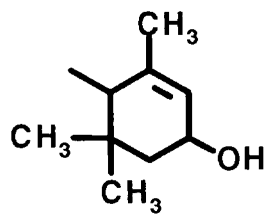
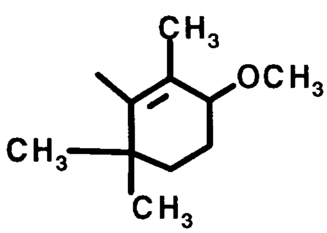
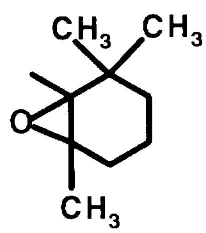
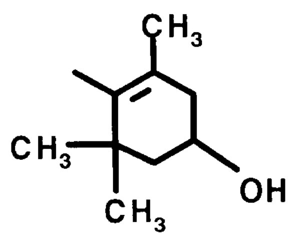
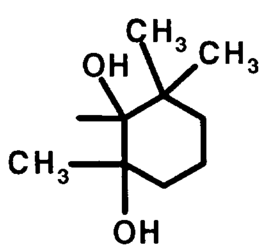
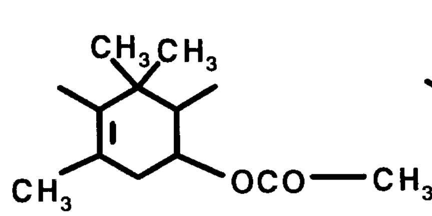
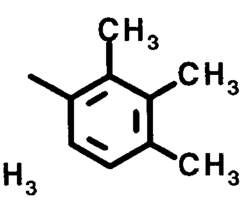

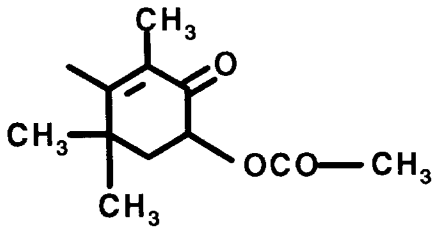 oder 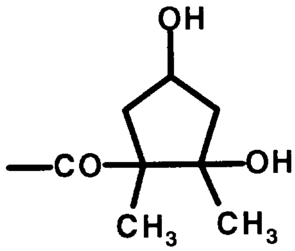

steht, mit N,N'-Carbonyldiimidazol bei 25 bis 70 °C in einem indifferenten Lösungsmittel, wie z.B. Tetrahydrofuran, Benzol, Toluol oder Chloroform und anschliessender Alkoholyse der gebildeten Imidazolide unter Zusatz einer katalytischen Menge eines Alkoholates mit einer Vitamin-E-Verbindung.

b) Bildung des Chlorides, bzw. des Bromides von einer Verbindung der Formeln (X), (XI) oder (XII) :

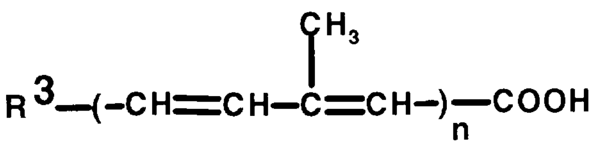

(X)

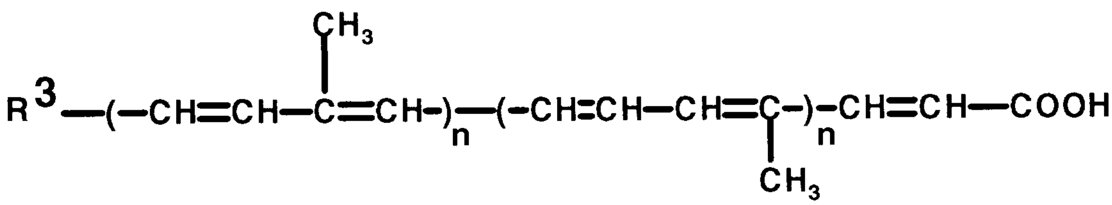

(XI)

$R^4$ —— COOH

(XII)

worin $R^4$ eine $C_1$- bis $C_{32}$-Alkyl- oder eine $C_2$- bis $C_{32}$-Alkenyl-, bzw. $C_2$- bis $C_{32}$-Alkapolyengruppe darstellt, mit einem Chlorierungsmittel, bzw. Bromierungsmittel wie z.B. Thionylchlorid, Oxalylchlorid oder Oxalylbromid und anschliessender Reaktion der entstandenen Chloride, bzw. Bromide mit einer Vitamin-E-Verbindung bei einer Temperatur von 40 bis 120 °C in einem indifferenten Lösungsmittel und in Gegenwart eines Katalyten.

Die *spontan dispergierbaren Konzentrate mit* Vitamin-E-Ester-Verbindungen haben überraschenderweise ***eine ausgezeichnete antitumorale Wirkung, sei es als Konzentrate, sei es als wässrige Mikroemulsionen.*** Solche Konzentrate ergeben, mit Wasser versetzt, Mikroemulsionen von hervorragender Phasenstabilität und von überaus deutlich erhöhtem Permeationsvermögen. Sie finden Verwendung als Arzneimittel, sowohl zur prophylaktischen, wie auch zur therapeutischen Verabreichung.

Die erfindungsgemässen, spontan dispergierbaren Konzentrate enthalten:

0,001 bis 15 Gewichts-% einzelner Ester von Vitamin-E-Verbindungen der Formeln (I) und (II), bzw. Kombinationen solcher Komponenten, sowie

0 bis 40 Gewichts-% eines als Hydrotrop, bzw. Co-Emulgator dienenden, pharmaverträglichen Lösungsmittels oder Lösungsmittelgemisches

0 bis 90 Gewichts-% eines pharmaverträglichen Tensides oder Tensidgemisches

0 bis 10 Gewichts-% eines Vitamins oder Provitamins

und gegebenenfalls übliche Trägerstoffe und/oder Verdünnungsmittel.

Die erfindungsgemäss einzusetzenden Tenside oder Tensidgemische können anionaktiv, kationaktiv, amphoter oder nicht-ionogen sein. Am besten sind sie *nicht-ionogen* und haben ein HLB-Verhältnis (d.h. eine "hydrophilic-lipophilic-balance") zwischen 2 und 18; bevorzugt liegt es zwischen 2 und 6 einerseits und 10 und 15 anderseits. HLB-Werte geben Auskunft über die hydrophilen und lipophilen Eigenschaften eines Emulgators. Vgl. dazu "Hydrophile-Lipophile Balance: History and recent Developments" von Paul

Becher im Journal of Dispersion Science and Technology. 5 (1), 81-96 (1984).

Geeignete anionische Tenside können sowohl sog. wasserlösliche Seifen als auch wasserlösliche synthetische Verbindungen sein.

Als Seifen eignen sich die Alkali-, Erdalkali- oder gegebenenfalls substituierten Ammoniumsalze von höheren Fettsäuren ($C_{10}$ bis $C_{22}$), wie z.B. die natürlichen Na- oder K-Salze der Oel- oder Stearinsäure, oder von natürlichen Fettsäuregemischen, welche sich u.a. aus Kokosnuss- oder Talgöl gewinnen lassen. Ferner sind als Tenside auch die Fettsäure-Methyltaurinsalze, sowie modifizierte und nicht-modifizierte Phospholipide zu erwähnen.

Häufiger werden jedoch sogenannte synthetische Tenside verwendet, insbesondere Fettsulfonate, Fettsulfate, sulfonierte Benzimidazol-Derivate oder Alkylarylsulfonate.

Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst. Beispiele hiefür sind das Na- oder Ca-Salz der Ligninsulfosäure, des Dodecylschwefelsäureesters und Sulfonsäuren von Fettalkohol-Äthylenoxyd-Addukten. Die sulfonierten Benzimidazol-Derivate enthalten vorzugsweise zwei Sulfonsäuregruppen und einen Fettsäurerest mit etwa 8 bis 22 C-Atomen. Alkylaryl-Sulfonate sind z.B. die Na-, Ca- oder Triäthanolaminsalze der Dodecylbenzolsulfonsäure, der Dibutylnaphthalinsulfonsäureoder eines Naphthalinsulfonsäure-Formaldehyd-Kondensationsproduktes.

Als nichtionische Tenside stehen in erster Linie zur Wahl die Polyglykolätherderivate von aliphatischen oder cycloaliphatischen Alkoholen, gesättigten oder ungesättigten Fettsäuren und Alkylphenolen, welche 3 bis 30 Glykoläthergruppen und 8 bis 20 C-Atome im (aliphatischen) Kohlenwasserstoffrest und 6 bis 18 C-Atome im Alkylrest enthalten können. Weiterhin kommen als nichtionische Tenside in Frage die wasserlöslichen, 20 bis 250 Äthylen-glykoläthergruppen und 10 bis 100 Propylenäthergruppen enthaltenden Polyäthylenoxydaddukte an Polypropylenglykol und Alkylpolypropylenglykol mit 1 bis 10 C-Atomen in der Alkylkette. Die genannten Verbindungen enthalten üblicherweise pro Propylenglykoleinheit 1 bis 5 Äthylenglykoleinheiten.

Als Beispiele nicht-ionischer Tenside seien erwähnt:

Nonylphenolpolyäthoxyäthanole, Ricinusölpolyglykoläther, Polypropylenpolyäthylenoxyd-Addukte, Tributylphenoxypolyäthoxyäthanol, Polyäthylenglykol und Octylphenoxypolyäthoxyäthanol. Ueberdies kommen auch Fettsäureester von Polyoxyäthylensorbitan, wie das PolyoxyäthylensorbitanTrioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorab als Halogenide, Methylsulfate oder Äthylsulfate vor, z.B. das Stearyltrimethylammoniumchlorid oder das Benzyl-di(2-chloräthyl)äthylammoniumbromid.

In hohem Masse bevorzugt zur Herstellung von erfindungsgemässen, spontan dispergierbaren Konzentraten sind *Phosphorsäureestertenside,* wie z.B. :

Tristyrylphenolpolyoxyäthylen-18-mono/dimethylphosphorsäureester

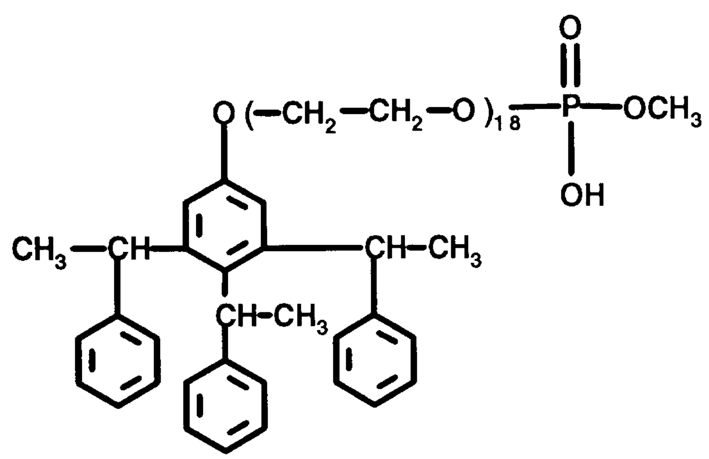

**(Soprophor® FL, Rhône-Poulenc)**

Nonylphenol-10-polyoxyäthylen-mono/dimethylphosphorsäureester

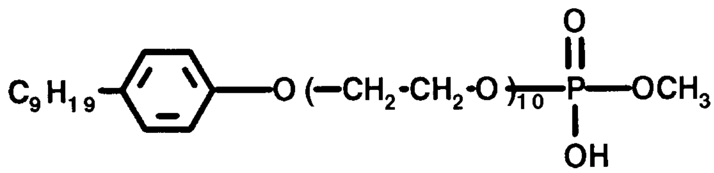

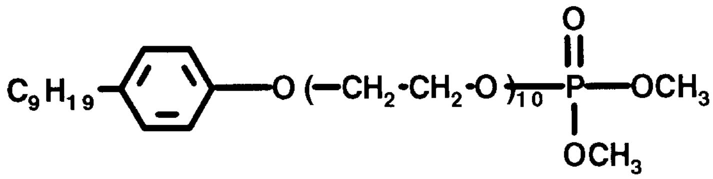

**(Diphasol® 3873, CIBA-GEIGY);**

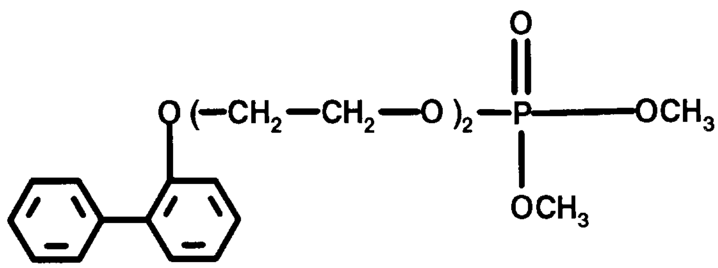

**(Tensid 508, CIBA-GEIGY);**

Tinovetin® JU (CIBA-GEIGY), ein Hydroxybiphenyl-10-Äthoxy-Phosphorsäureester;
Butyl-mono-4-Äthoxy-Phosphorsäureester (Zerostat® AT, CIBA-GEIGY), bzw.

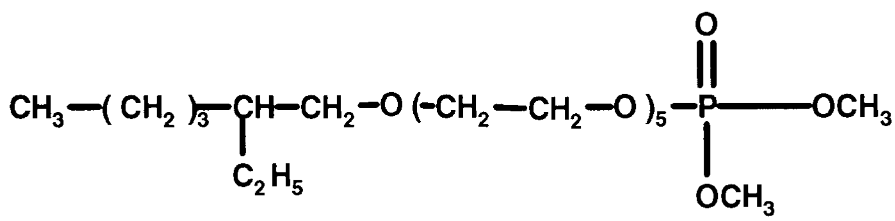

**(Zerostat ® AN , CIBA-GEIGY)**

Als *Hydrotrop,* bzw. *Co-Emulgator* dienende, pharmaverträgliche Lösungsmittel lassen sich einsetzen, z.B.:
Ester eines aliphatischen Alkohols ($C_3$ bis $C_{18}$) mit einer aliphatischen Carbonsäure ($C_{10}$ bis $C_{22}$), wie etwa Isopropyllaurat, Hexyllaurat, Decyllaurat, Isopropylmyristat, Isopropylpalmitat und Laurylmyristat; Kohlenwasserstoffe mit einer geraden Kohlenstoffkette ($C_{12}$ bis $C_{32}$), welche mit 6 bis 16 Methylgruppen substituiert ist und 1 bis 6 Doppelbindungen aufweisen kann, wofür Terpene wie Polymethylbutane und Polymethylbutene als Beispiele dienen mögen.
Mono-Ester aus Äthylenglykol oder Propylenglykol mit einer aliphatischen Carbonsäure ($C_6$ bis $C_{22}$), wie etwa Propylenglykolmonolaurat und Propylenglykolmonomyristat.
Ester aus einem aliphatischen Alkohol ($C_{12}$ bis $C_{22}$) mit Milchsäure, wie z.B. Myristyl- oder vorzugsweise Lauryl-Lactat; Mono-, Di- oder Triester des Glycerins mit einer aliphatischen Carbonsäure ($C_6$ bis $C_{22}$), wie z.B. Glyceryl-Caprylat, oder Miglyol® 812 Neutralöl (Oleum neutrale).
Ester aus einem Poly(2-7)äthylenglykolglyzerinäther mit mindestens einer freien Hydroxylgruppe mit einer aliphatischen Carbonsäure ($C_6$ bis $C_{22}$), wie z.B. aliphatische Alkohole ($C_{12}$ bis $C_{22}$), somit u.a. Dodecanol, Tetradecanol, Oleylalkohol, 2-Hexyldecanol und 2-Octyldecanol.
Ester mit mindestens einer freien Hydroxylgruppe, aus Poly-(2-10)glykol mit einer aliphatischen Carbonsäure ($C_6$ bis $C_{22}$), Monoäther aus einem Polyäthylenglykol mit einem aliphatischen Alkohol ($C_{12}$ bis $C_{18}$), wie z.B. Polyoxyäthylen ($C_{10}$)-octyläther.

Heterocyclische Verbindungen, wie z.B. 1-Methyl-2-Pyrrolidon.

Alle technischen Tenside wurden vor dem Eintrag in die spontan dispergierbaren Konzentrate mittels Filtration, bzw. Chromatographie über neutralem Aluminiumoxyd mit einem inerten Lösungsmittel wie z.B. Tetrahydrofuran, Äthylalkohol oder Methylenchlorid gereinigt.

Als Zusätze in die erfindungsgemässen spontan dispergierbaren Konzentrate eignen sich Vitamine und Provitamine (wie z.B. Vitamin A, Retinol, Carotine, Tocopherole), sowie Antioxydantien und Radikalfänger.

Die zur pharmazeutischen Anwendung erforderliche Tagesdosis beträgt 0,001 bis 25 mg/kg Körpergewicht, wenn möglich verteilt auf 2 bis 3 Einzeldosen. Hiefür lassen sich die KONZENTRATE mit Vitamin-E-Esterverbindungen in die gängigen pharmazeutischen Zubereitungen und Darreichungsformen wie Dragees, Tabletten, Kapseln, Pulver, Granulat, Pellets, Lösungen, Ampullen, Emulsionen, Crèmes oder Zäpfchen zusammen mit den üblichen Trägerstoffen und/oder Verdünnungs- und Stabilisierungsmitteln einarbeiten.

Die Gegenstand der Erfindung bildenden spontan dispergierbaren Konzentrate, welche Wirkstoffe oder Wirkstoffmischungen der Formeln (I) und (II) enthalten, können dem Menschen oral, durch Injektion (intravenös, subkutan oder intramuskulär) oder in anderer Weise verabreicht werden. Wenn sie als feste Darreichungsformen für die orale Verwendung aufbereitet werden, kann dies in Form von Tabletten, Granulaten, Pellets, Pulvern oder Kapseln, usw. geschehen. Die Aufbereitungen können Zusatzstoffe enthalten, z.B. einen Arzneimittelträger wie eine Saccharid- oder Cellulose-Grundlage, ein Bindemittel wie Stärkepaste oder Methyl-Cellulose, ein Füllmittel oder ein Desintegriermittel, usw. - wobei Zusatzstoffe eingesetzt werden, wie sie üblicherweise bei der Herstellung medizinischer oder paramedizinischer Formulierungen verwendet werden. Wenn die erfindungsgetreuen Konzentrate als flüssige Darreichungsformen zu oraler Verabreichung gelangen, so können sie irgend eine aus wässrigen Zubereitungen für innere Verwendung, aus Suspensionen, Emulsionen und Sirups usw. ausgewählte Form haben, und sie können ausserdem in der Form getrockneter Präparate vorliegen, welche vor ihrer Verwendung in Lösung oder Emulsion gebracht werden.

Wenn die erfindungsgemässen Konzentrate in der Form wässriger Lösungen, Suspensionen oder öliger, bzw. wässriger Emulsionen, vorzugsweise als Mikroemulsionen aufbereitet sind, können sie auch injiziert werden. Die Injektionslösungen werden jedoch üblicherweise kurz vor der Anwendung hergestellt, indem man die Konzentrate in wässrigen, flüssigen Medien wie sterilem Wasser, Glucoselösung oder physiologischer Kochsalzlösung auflöst oder suspendiert und entgast.

Falls erforderlich, können zu einem Injektionspräparat üblicherweise verwendete Lösungsmittel, Stabilisierungsmittel, Konservierungsmittel und Zusätze für die Herstellung isotonischer Lösungen hinzugegeben werden. Die auf diese Weise erhaltenen Injektions-Präparate werden intravenös, intramuskulär, subkutan oder in einer anderen geeigneten Weise verabreicht.

Die vorliegende Erfindung betrifft ebenfalls ***pharmazeutische Präparate,*** welche die beschriebenen, spontan dispergierbaren Konzentrate/Ultramikroemulsionen zur Bekämpfung des Wachstums von Tumorzellen enthalten. Bei den der Erfindung entsprechenden pharmazeutischen Präparaten handelt es sich um solche zur enteralen (wie oralen oder rektalen), sowie zur parenteralen oder topischen Verabreichung an Warmblüter, welche das spontan dispergierbare Konzentrat allein oder zusammen mit einem pharmazeutisch anwendbaren Trägermaterial enthalten.

Die Dosierung der erfindungsgemässen Konzentrate/Mikroemulsionen hangt von der Warmblüterspezies, dem Alter und dem individuellen Zustand, sowie von der Verabreichungsart ab. So werden z.B. zur Erzielung eines Abtötungseffektes von Tumorzellen an Warmblütern mit geringem Körpergewicht, wie z.B. Mäusen, Ratten und Hamstern, bei sukutaner Verabreichung Dosen im Bereich von ca. 0,1 bis 50 mg/kg Körpergewicht, und bei intraperitonealer Verabreichung Dosen im Bereich von 0,05 bis 5 mg/kg Körpergewicht angewandt.

Die oralen und rektalen Formen der neuen pharmazeutischen Präparate enthalten zwischen 1 bis 95 %, vorzugsweise zwischen 10 bis 95 %, insbesondere zwischen 20 bis 95 % des erfindungsgemässen, spontan dispergierbaren Konzentrates. Sie können z.B. in Dosis-Einheitsform vorliegen, also als Dragées, Micropellets, Tabletten, Suppositorien oder Ampullen und vor allem als Kapseln.

Geeignete pharmazeutisch anwendbare Trägerstoffe für die oralen Formen sind hauptsächlich Füllstoffe, wie Zucker (z.B. Lactose, Saccharose, Mannit oder Sorbit), Cellulosepräparate und/oder Calciumphosphate (z.B. Tricalcium- oder Calciumhydrogenphosphat), ferner Bindemittel wie Stärke-kleister unter Verwendung von u.a. Mais-, Weizen-, Reis- oder Kartoffel-stärke, Gelatine, Traganth, Methylcellulose, Hydroxyl-Methylcellulose, Hydroxypropyl-Methylcellulose, Natriumcarboxymethylcellulose und/oder Polyvinylpyrrolidon und/oder Sprengmittel (wenn erwünscht), wie die obgenannten Stärken, ferner Carboxymethylstärke, quervernetztes Polyvinylpyrrolidon, Agar, Alginsäure oder ein Salz davon, wie z.B. Natriumalginat.

Als Fliessreguliermittel sind z.B. die Polyäthylenglykole Nr. 200 bis 600 und höher geeignet.

Die beim Menschen als Darreichungsform bevorzugten Gelatinekapseln werden mit geeigneten Ueberzügen versehen, wobei man u.a. konzentrierte Zuckerlösungen [welche gegebenenfalls arabischen Gummi, Talk, Polyvinylpyrrolidon, Polyäthylenglykol und/oder Titandioxid enthalten] Lacklösungen (wässrige oder solche, die unter Verwendung organischer Lösungsmittel aufbereitet worden sind), oder magensaftresistente Ueberzüge aus Lösungen von geeigneten Cellulosepräparaten, wie mikrokristalliner Cellulose (Avicel®), Acetylcellulosephthalat, Hydroxymethylcellulosephthalat (Metolose™), Hydroxypropylmethylcellulose-Acetatsuccinat (AQOAT™) oder einem Copolymerisat wie Eudragit® L 30 D verwendet.

Als erfindungsgemäss besonders geeignete, oral anwendbare pharmazeutische Darreichungsform eignen sich Steckkapseln aus Gelatine und einem Weichmacher, wie Glyzerin oder Sorbitol. Die Weich- bzw. Hartgelatine-Kapseln, sowie solche aus AQOAT™ (Hydroxypropyl-Methylcellulose-Acetat-Succinat) können das der Erfindung entsprechende, spontan dispergierbare Konzentrat im Gemisch mit Füllstoffen, wie Laktose, Bindemitteln wie Stärke und/oder Gleitmitteln wie Talk oder Magnesium-Stearat und gegebenenfalls mit Stabilisatoren und Antioxydantien wie z.B. $\alpha$-, $\beta$- oder $\gamma$-Tocopherol enthalten. Der Einsatz von geeigneten Flüssigkeiten wie flüssigen Polyäthylenglykolen No. 200 bis 600 als Verdünnungsmittel kann sich empfehlen, wobei sich ebenfalls Stabilisatoren und Antioxydantien zufügen lassen.

Zur parenteralen Verabreichung werden die erfindungsgemässen Konzentrate mit destilliertem Wasser versetzt. Der entstehenden wässrigen Injektions-Mikroemulsion können viskositätserhöhende Stoffe, wie z.B. Na-Carboxymethylcellulose, Sorbit, Mannit und/oder Dextran, und gegebenenfalls auch Stabilisatoren und Antioxydantien zugefügt werden.

Die pharmazeutischen Präparate für die parenterale Anwendung enthalten vorzugsweise zwischen 0,1 bis 60 %, und hauptsächlich zwischen 1 bis 40 % des erfindungskonformen, spontan dispergierbaren Konzentrates.

Als topisch anwendbare Präparate, welche sich vornehmlich zur Prophylaxe und Therapie von Hautkrebsarten eignen, kommen z.B. Crèmen, Salben, Pasten, Pomaden, Schäume, Tinkturen und Lösungen in Betracht, welche zwischen 0,01 bis 70 % des erfindungsgemässen Konzentrates enthalten.

Für Crèmen und Oel-in-Wasser-Emulsionen, welche mehr als 50 % Wasser aufweisen, verwendet man als oelige Grundlage in erster Linie Fettalkohole, z.B. Lauryl-, Cetyl- oder Stearylalkohol, flüssige bis feste Wachse, z.B. Isopropylmyristat, Woll- oder Bienenwachs und/oder Kohlenwasserstoffe wie z.B. Vaseline (Petrolatum) oder Paraffinoel. Zur Emulgierung dieser öligen Grundlagen eignen sich in erster Linie oberflächenaktive, pharmaverträgliche Substanzen mit vorwiegend hydrophilen Eigenschaften, wie z.B. nicht-ionogene Emulgatoren, vorab Fettsäureester von Polyalkoholen oder Äthylenoxydaddukten (etwa Polyglycerinfettsäureester oder Polyäthylensorbitan-Fettsäureester) mit einem HLB-Wert von unter 8. Zusätze zur Wasserphase sind u.a. Mittel, welche die Austrocknung der Crèmen vermindern, z.B. Polyalkohole wie Glyzerin, Sorbit, Propylenglykol und/oder Polyäthylenglykole No. 200 bis No. 600, ferner Konservierungsmittel, Riechstoffe, etc.

Salben sind Wasser-in-Oel Emulsionen, die bis zu 70 %, vorzugsweise jedoch zwischen 20 und 50 % Wasser oder wässrige Phasen enthalten.

Als Fettphase kommen in erster Linie Kohlenwasserstoffe, z.B. Vaselin, Paraffinoel und/oder Hartparaffine in Frage, welche zur Verbesserung des Wasserbindungsvermögens geeignete Hydroxydverbindungen, wie z.B. Fettalkohole oder Ester, davon etwa Cetylalkohol oder Wollwachsalkohole, enthalten.

Fallweise werden noch Emulgatoren mit einem HLB-Wert von 8 bis 16, wie z.B. Sorbitan-Fettsäureester (etwa Sorbitanisostearol) zugesetzt. Zusätze zur Wasserphase sind u.a. Feuchthaltungsmittel, wie Polyalkohole (Glycerin, Propylenglykol, Sorbit und/oder Polyäthylenglykole No. 200, 400, 600); ferner Konservierungsmittel, Riechstoffe, etc.

Fettsalben sind wasserfrei und enthalten als Grundlage vornehmlich Kohlenwasserstoffe, z.B. Paraffin, Vaselin und/oder flüssige Paraffine; überdies natürliche oder partial synthetische Fette wie z.B. Kokosfettsäuretriglycerid, ferner: Fettsäurepartialester des Glycerins, wie z.B. die im Zusammenhang mit den Salben erwähnten, die Wasser-Aufnahmefähigkeit steigernden Fettalkohole, Emulgatoren und/oder Zusätze.

Pasten sind Crèmen und Salben mit sekretabsorbierenden Puderbestandteilen, wie beispielsweise Metalloxide (etwa Titanoxid oder Zinkoxid), ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende Oel-in-Wasser Emulsionen der erfindungsgemässen, spontan dispergierbaren Konzentrate, wobei halogenierte Kohlenwasserstoffe (wie z.B. Chlorfluorniederalkane: etwa Dichlordifluormethan und Dichlortetrafluoräthan) als Treibmittel verwendet werden. Dazu kommen gegebenenfalls die üblichen Zusätze wie Konservierungsmittel, usw.

*Verfahrensbeispiele zur Herstellung von Estern mit Vitamin–E–Verbindungen*

*1. Herstellung von DL–α–Tocopherylpalmitat*

*Zu 450 mg* DL-α-Tocopherol und 50 mg Dimethylformamid in 30 ml Toluol werden bei 20 °C 350 mg Palmitoylchlorid (ca. 20 % Überschuss) in 20 ml Toluol zugetropft.
Die Reaktionslösung wird am Rückfluss während drei Stunden auf 80 °C erhitzt. Anschliessend wird das Lösungsmittel durch Vakuumdestillation entfernt und der Rückstand in Acetonitril umkristallisiert. Man erhält das reine *DL–α–Tocopherylpalmitat* mit einem Schmelzpunkt von $53^{8}$ bis $55^{2}$ °C.

Auf gleiche Weise werden auch folgende Verbindungen hergestellt:

| | | |
|---|---|---|
| DL-α-Tocopherylpivaloylat | BI | 1.50102 |
| DL-α-Tocopherylcrotonat | BI | 1.50378 |
| DL-α-Tocopheryl-nValerat | BI | 1.49528 |
| DL-α-Tocopherylcaproylat | UV λmax. 296,8 | |
| | BI | 1.48018 |
| DL-α-Tocopheryl-10-Undecenoat | UV λmax. 296,6; 233,6 | |
| | BI | 1.49188 |
| DL-α-Tocopheryl-2-Dodecenoat | UV λmax. 296,4; 233,0 | |
| | BI | 1.49388 |
| DL-α-Tocopheryl-Oleat | BI | 1.48158 |

*2. Herstellung von DL–α–Tocopheryl–Linolenat*

Zu 300 mg Linolensäure in 20 ml Toluol tropft man bei 10 °C 200 mg Oxalylchlroid in 30 ml Toluol.
Die Reaktionslösung wird bei 20 °C während 24 Stunden stehen gelassen.
Anschliessend werden unter Vakuum 10 ml Toluol abdestilliert und dann 400 mg DL-α-Tocopherol, sowie 50 mg Dimethylformamid zugesetzt. Nach 2-stündiger Erhitzung bei 90 °C wird das Lösungsmittel durch Vakuumdestillation entfernt. Der Rückstand wird auf einer Silicagelsäule mit Cyclohexan/Essigester (80:20) als Eluiermittel chromatographiert. Man erhält da*s DL–α–Tocopheryllinolenat* mit einem Brechungsindex BI von 1.48290.

Auf entsprechende Weise werden auch folgende Verbindungen hergestellt:

| | | | |
|---|---|---|---|
| DL-α-Tocopheryloleat | | BI | 1.49302 |
| DL-α-Tocopheryllinolat | | BI | 1.48888 |
| DL-α-Tocopheryl-all trans-Retinat | | BI | 1.55350 |
| | IR | 1723 $cm^{-1}$ | ν (C=O) |
| | NIR | 1583 $cm^{-1}$ | (C=C) [Retinat] |
| DL-α-Tocopheryl-13-cis-Retinat | | | |
| | NIR | 1583 $cm^{-1}$ | (C=C) [Retinat] |

N.B.: BI = Brechungsindex

$$n_D^{20} = \textbf{Refractive Index (RI)},$$

gemessen im DUR Refractometer Schmidt + Haensch, Berlin
IR = Infrarot Spektren, gemessen am Spektrophotometer Perkin-Elmer 683G
NIR = Near infrared (FT Raman) Spektren, gemessen am Spektrometer BRUKER IFS 88 mit Raman Modul FRA 106 und NIR Neodym-YAG-Laser, Anregung bei 1064 nm

UV-Spektren gemessen am Spektrophotometer Shimadzu UV-160A

*Zusammensetzungsbeispiele von erfindungsgemässen, spontan dispergierbaren Konzentraten zur Verwendung als Arzneimittel, welche als antitumorale Wirkstoffe Vitamin–E–Ester gemäss den Formeln (I) und (II) enthalten*

a) 0,5 bis 15 Gewichts-% eines oder mehrerer Ester mit Vitamin-E-Verbindungen der Formeln (I) und (II)
0 bis 40 Gewichts-% Isopropylmyristat, Isopropylpalmitat oder Miglyol® 812 (Dynamit Nobel)
20 bis 45 Gewichts-% Diphasol® 3873 (CIBA-GEIGY)
20 bis 45 Gewichts-% Invadin® JFG 800 % (CIBA-GEIGY)
b) 0,5 bis 15 Gewichts-% eines oder mehrerer Ester mit Vitamin-E-Verbindungen der Formeln (I) und (II)
0 bis 40 Gewichts-% Isopropylmyristat, Isopropylpalmitat oder Miglyol® 812 (Dynamit Nobel)
20 bis 45 Gewichts-% Invadin® JFG 800% (CIBA-GEIGY)
20 bis 45 Gewichts-% Soprophor® FL (Rhône-Poulenc)

Miglyol® 812-Neutralöl ist ein *Neutralöl* (Oleum neutrale) der Firma Dynamit Nobel, das einem gemischtsäurigen Triglycerid der fraktionierten Kokosfettsäuren $C_8$ bis $C_{10}$ entspricht.

Diphasol® 3873 ist ein Mischemulgator, bestehend aus je 50 % der beiden Verbindungen mit den Formeln:

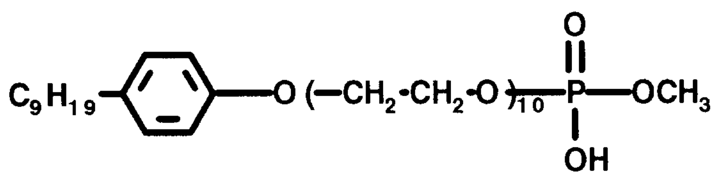

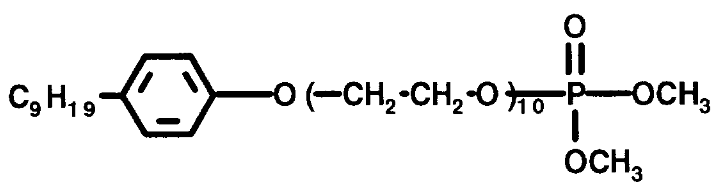

**(DIPHASOL® 3873 CIBA-GEIGY)**

Invadin® JFG 800% (CIBA-GEIGY) ist ein tert. Octylphenylpolyoxyäthylenaether mit 9 bis 10 Oxyäthylengruppen.

Soprophor® FL (Rhône-Poulenc) ist ein Tristyrylphenolpolyoxyäthylen-18-mono/dimethyl-Phosphorsäureester:

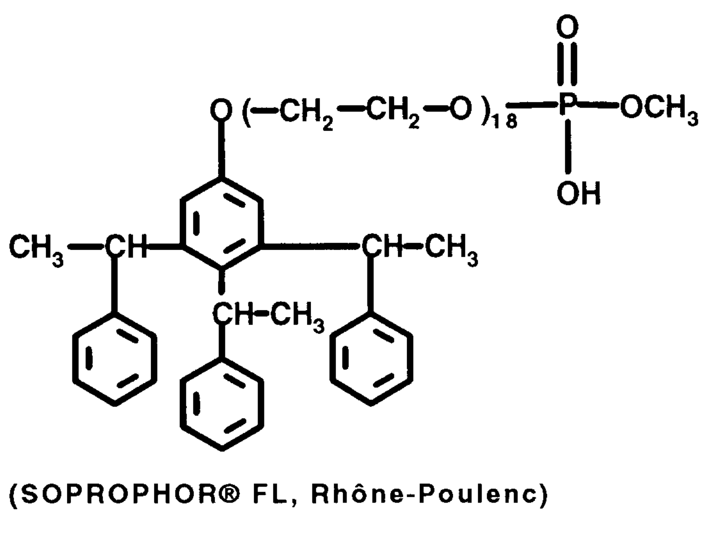

**(SOPROPHOR® FL, Rhône-Poulenc)**

*Experimenteller Nachweis des Spreitungs– und Wanderungsvermögens der erfindungsgemässen MARIGENOL®–Konzentrate und Ultramikroemulsionen.*

Methode: DG-Platte 0,25 mm Kieselgel 60F254, Merck Art. No. 11'798 mit Konzentrationszone
Laufmittel: PBS Dulbecco's ohne Ca und Mg (= Ringerlösung, bzw. Kochsalzlösung, gepuffert)
Entwicklungszeit: 15 Minuten

| ***ERGEBNIS** : Rf.– Werte* | | |
|---|---|---|
| | 0,1 % Substanz, gelöst in Methylenchlorid | Microemulsion mit 1000 ppm. Konzentrat |
| | Rf.-Werte: | |
| Ergocalciferyl-all trans Retinat : (mit DL-α-Tocopheryl-Acetat als Co-Emulgator) | 0 | 45,45 |
| DL-α-Tocopheryl-C 8:0 Ester | 0 | 21,21 |
| DL-α-Tocopheryl-C 10:1-Undecenoat | 0 | 59,68 |
| DL-α-Tocopheryl-C 16:0 (Palmitat) | 0 | 48,48 |

*P.S.:* Mikroemulsionen 1:1'000 = 1'000 ppm Konzentrat = 1 mg/ml.
Die oben angeführten Rf.-Werte vermitteln wichtige Hinweise auf das Verhalten der erfindungsgemässen, spontan dispergierbaren Konzentrate, welche mit Ester-Verbindungen der Formeln (I) und (II) gebildet und mit Wasser verdünnt werden, in den Zellverbänden und insbesondere an den Zellmembranen. Durch die geringe Oberflächenspannung für Mikroemulsionen aus diesen Konzentraten mit Werten von 30 bis 32 $mN/m^{-1}$, den kleinen Teilchenradius der sich ausbildenden Mikromizellen, sowie die niedrige Viskosität der Emulsionen werden vorab das Diffusionsvermögen und die Spreitung (und damit die Bioverfügbarkeit) der antitumoral wirksamen Verbindungen der Formeln (I) und (II) in ausnehmend günstiger Weise beeinflusst. Kontrollmessungen, ausgeführt am Institut für Polymere der E.T.H., Zürich, haben ergeben, dass die hydrodynamischen Radien für die Mizellen um 1,5 bis 3 nm betragen. (Prof. Dr. Pier Luigi LUISI und Prof. Dr. Peter SCHURTENBERGER).

*Beispiel für die pharmazeutische Herstellung eines Systempräparates mit erfindungsgemässen Konzentraten in der Form von "multiple units".*

a) Granulierung

| | |
|---|---|
| Metolose® 90 SH-4000 (Shin-Etsu Chemical) | 90.0 g |
| Avicel® PH-101 | 80.3 g |
| Erfindungsgemässes KONZENTRAT | 139.4 g |
| Aerosil® 200 | 80.3 g |
| Σ | 390.0 g |

Granulieren/formen im Schnellmixer oder im Rotationsbett unter Zusatz von 110g Äthanol, brechen, sieben 18 bis 42 mesh, trocknen 24 h bei 40 °C.
b) MSR- und RETARD-Ausrüstung
im Rotationsbett mit AQOAT® AS-HG (Shin-Etsu Chemical) und Talk
c) Zusammensetzung fertiges Granulat/bzw. Micropellets

| | |
|---|---|
| Kernmaterial | 44 % |
| Erfindungsgemässes KONZENTRAT | 25 % |
| MSR-Beschichtung | 31 % |
| Σ | 100 % |

N.B.: MSR = Magensaft-Resistenz. Die Pellets/Granulate gemäss a) können auch ohne Befilmung unmittelbar in Kapseln abgefüllt werden, welche aus AQOAT™ (HPMC-AS-M oder HPMC-AS-N) hergestellt sind, mit Aceton/Ethanol 1:1 verschlossen werden und so die Funktionen der MSR und der verzögerten Abgabe (Retard) angemessen steuern.

*Biologische Prüfungen*

Die *antitumorale Wirkung* von spontan dispergierbaren Konzentraten, welche Vitamin-E-Ester gemäss den Aufarbeitungsbeispielen a) und b) enthalten, bzw. jene der damit gebildeten Mikroemulsionen wird anhand folgender Prüfungsergebnisse bestätigt:

*1. In vitro–Tests mit geeigneten Tumorzell–Linien*

Es wurde ein biologisches Assay-System entwickelt, das mit Mikrotiterplatten und Verdünnungsreihen arbeitet. Angesetzt werden je $10^4$/ml Tumorzellen in Kulturmedium RPMI 1640 mit 10 % fötalem Kalbserum inaktiviert (GIBCO); sie werden so undicht ausgesät, dass sie während des Assays wachsen können, in sog. nichtkonfluenten Monolayers. Die Probenzugabe erfolgt nach 6 bis 24 Stunden, mit 100 µl pro Reihe, die man im 1. Loch mit 100 µl Medium versetzt. Davon wird die Hälfte entnommen und in das folgende Loch eingebracht, wieder mit 100 µl Medium versetzt, usf. Es entsteht eine geometrische Verdünnungsreihe $n\frac{1}{2}$.

Die Proben werden im Plaque Assay während 3 bis 5 Tagen bei 37° C mit $3\frac{1}{2}$ % $CO_2$ inkubiert. Anschliessend färben/fixieren mit 0,1 % Kristallviolett (Fluka, Buchs) in einer Lösung von 70 % Methanol, 1 % Formaldehyd, 29 % Wasser. Die Auswertung wird am Mikroskop vorgenommen, Vergrösserung 300-fach. Man bestimmt die grösste cytotoxische Verdünnung. Die quantitative Auswertung lässt sich auch mittels Scanning und Absorptionsmessung am Spektrophotometer vornehmen.

*AUSWERTUNG DER ERGEBNISSE*

a) TS/A-Assay: grösste zelltoxische Verdünnung

| **TUMORLINIE**<br>**PRÄPARAT** | **TS/A**<br>**Murines Adenokarzinom**<br>**In Verdünnung wirksam bis 1:** | |
|---|---|---|
| **EXPOSITION** | **24 h** | **72 h** |
| **C 8 : 0-DL-α-TOCO-PHERYLESTER** | **4'000'000** | **32'000'000** |

**TS/A :murines Adenokarzinom (spontanes Mammakarzinom)**
**Prof.Dott. Guido FORNI, Istituto di Microbiologia,**
**Università degli Studi di TORINO, Scuola di Medicina**

b) Py 6-ASSAY: grösste zelltoxische Verdünnung

| TUMORLINIE<br><br>PRÄPARAT | Py 6 (Polyoma virus transformed 3T3 mouse cells, Fibroblasten) In Verdünnung wirksam bis 1 : | |
|---|---|---|
| EXPOSITION | 20 h | 60 h |
| C 8 : 0-DL-α-TOCO-PHERYLESTER | 8'000'000 | 16'000'000 |
| C 11 : 1-DL-α-TOCO-PHERYLESTER | 12'000'000 | 48'000'000 |
| C 18 : 1-DL-α-TOCO-PHERYLESTER | 6'000'000 | 24'000'000 |
| C 18 : 3-DL-α-TOCO-PHERYLESTER | 6'025'000 | 48'200'000 |
| DL-α-TOCOPHERYL-all trans-RETINAT | 12'000'000 | 96'000'000 |

Py 6 : Polyoma-Virus-transformierte 3T3 Zellen der Maus (Fibroblasten)

c) Andere Tumor-Zell-Linien

| TUMORLINIEN<br>PRÄPARAT | Y T 43<br>In Verdünnung wirksam bis 1 : | P 815<br>In Verdünnung wirksam bis 1 : | L 929<br>In Verdünnung wirksam bis 1 : |
|---|---|---|---|
| Exposition | 4 Tg | 4 Tg | 2 Tg |
| C 8 : 0-DL-α-TOCOPHERYLESTER | 32'000'000 | 32'000'000 | 64'000'000 |
| C 16 : 0-DL-α-TOCOPHERYLESTER | 16'000'000 | 16'000'000 | 64'000'000 |

Y T 43 : ibridoma/anticorpi monoclonali (Hybridom-Zellen)
P 815 : mastocitoma
L 929 : fibroblastoma

Prof. Guido Forni, Istituto di Microbiologia, Università degli Studi di Torino, Scuola di Medicina.
d) Zelltoxizität auf humanen Tumorzell-Linien
Ueberiebensquote in % (MTT-Methode mit Tetrazolblau)
VERDUENNUNG 1 : 1'000'000

| ZELL-LINIE<br>PRÄPARAT | ST-4 | RAJI | K 562 | H L 60 |
|---|---|---|---|---|
| DL-α-TOCOPHERYL-CAPROYLAT | 1 | 8 | 7 | 7 |
| C 11 : 1-DL-α-TOCOPHERYLESTER | -- | 1 | 1 | 0 |

VERDUENNUNG 1: 10'000'000

| ZELL-LINIEN PRÄPARAT | ST-4 | RAJI | K 562 | H L 60 |
|---|---|---|---|---|
| TOCO-CAPROYLAT | 41 | 73 | 85 | 56 |
| DL-α-TOCOPHERYL-C 11:1 | 67 | 10 | 85 | 100 |

**ST-4 Linfoma convoluto T** **K 562 Eritroleucemia**
**RAJI Leucemia linfoide B** **H L 60 Leucemia mieloide**

Weitere *Prüfungen mit humanen Tumorzell-Linien*

Das BATTELLE INSTITUT, Frankfurt, (Dr. Matthias GIESE) hat die zytotoxische Wirkung bei verschiedenen soliden, eher langsam wachsenden Tumorarten geprüft. Eingesetzt wurden 2 %-Konzentrate (gewichtsmässig) von:

A CALCIOL-CHOLECALCIOL-ESTER-MISCHUNG
B CALCIOL-all trans-RETINAT
C DL-α-TOCOPHERYLESTER-MISCHUNG

mit folgenden humanen Tumorzell-Linien (DKFZ, Heidelberg, BRD):

| | |
|---|---|
| 1 EJ 28 | Blasen-Karzinom |
| 2 LX-1 | Lungen-Karzinom |

Als Kontroll-Substanzen wurden biologische "response modifiers" (BRM) verwandt:

a rhu Interferon-Gamma (Biozol, BRD)
b rhu Tumor-Nekrose Faktor Alpha (Biozol, BRD)

Ergebnis-Ausweis mittels Verdünnungsreihen und in der Kurzform der $IC_{50}$. (= Inhibitory Concentration; bzw. LC = Lethal Concentration) Inkubierung der Zellen über 24 h, bzw. 72 h bei 37 °C; Vitalitäts-Färbetest nach 3, bzw. 14 Tagen. Testbereich $1:10^5$ bis $1:10^9$.

Es konnten kleinere Unterschiede zwischen den Zell-Linien, wie auch zwischen den drei Wirkstoff-Konzentraten festgestellt werden. Die $LC_{50}$ lag im Verdünnungsbereich $1:10^6$ bis $1:10^7$.

Der Hauptbefund der Tests lautet: *es liegt eine zytotoxische und keine zytostatische Wirkung vor.*

Der stärkste Zytotoxizitäts-Effekt liess sich für die CALCIOL/CHOLECALCIOLESTER-MISCHUNG ermitteln. Aber auch die Verwendungen eines Konzentrates mit einer Mischung von Tocopheryl-Estern ergab beachtliche Resultate und kann für die abwechselnde Verabreichung in Frage kommen. Die Kontroll-Substanzen BRM zeigten hingegen keine messbaren Wirkungen auf die 2 geprüften Zell-Linien.

Die nachstehend als Beilage 1/1, Abbildung 1 und 2 wiedergegebenen graphischen Darstellungen fassen die Ergebnisse für die drei Konzentrate A, B und C zusammen. Vgl. die techn. Beilage. Abbildungen 1 + 2.

*Nachweis der Membran-Penetration an der Tumorzelle*

Am Lichtmikroskop wie auch mithilfe der "Laser scanning microscopy" lässt sich aufzeigen, dass wenige Stunden nach der Inkubation (Beispiel Py6 - Polyoma Virus transformierte Fibroblasten, 3T3-Zellen der Maus; dünn ausgesät, mittlere Verdünnung der Wirkstoff-Konzentrate) sich ein Kranz von Vakuolen um den Zellkern herum ausbildet.

Der analytische Nachweis, dass diese Vakuolen die Vitamin-E-Wirksubstanzen enthalten, kann sehr deutlich dadurch erbracht werden, dass man den gereinigten inkubierten Tumorzellen das Zellplasma entnimmt, es zentrifugiert und mithilfe von mizellarer Kapillar-Zonen-Elektrophorese untersucht, (Beckman Instruments, P/ACE System 2000, Version 1.50). Es entsteht nach rund 6 Minuten Laufzeit ein scharfer, charakteristischer Peak.

**Patentansprüche**

**1.** Spontan dispergierbares Konzentrat, welches mit Wasser versetzt Mikroemulsionen von hervorragender Phasenstabilität und von überaus deutlich erhöhtem Permeationsvermögen ergibt, dadurch gekennzeichnet, dass es als antitumorale Komponente
0,001 bis 15 Gewichts-% einer Verbindung der Formeln (I) und (II):

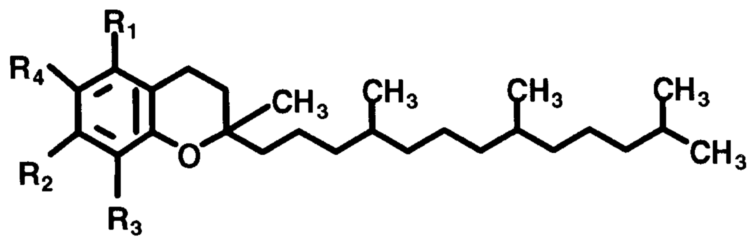

(I)

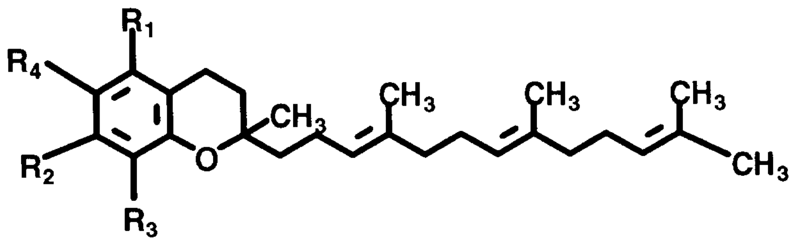

(II)

wobei die Radikale $R_1$, $R_2$ und $R_3$ Wasserstoff oder Methyl bedeuten und das Radikal $R_4$ eine $C_1$- bis $C_{32}$-Alkyl, bzw. eine $C_2$- bis $C_{32}$-Alkenyl- oder Alkapolyenkette mit funktioneller Carboxylgruppe oder eine Gruppe der Formeln (VII) oder (VIII) :

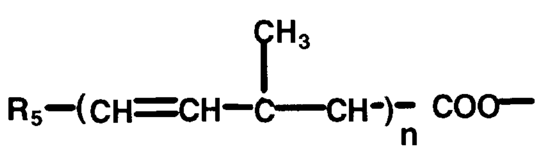

(VII)

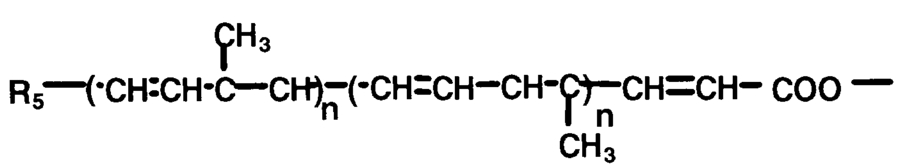

(VIII)

darstellt, worin n die Zahlen 1, 2, 3, 4 oder 5 bezeichnet und $R_5$ für ein Radikal der Formeln

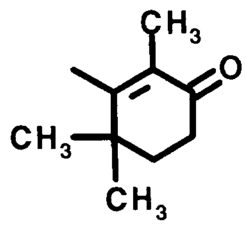
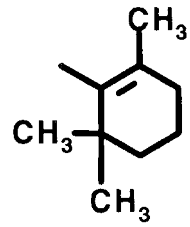
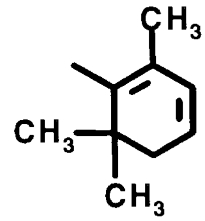

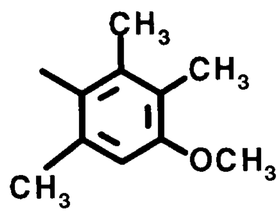
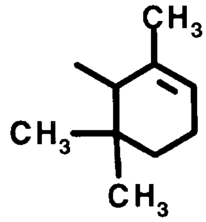
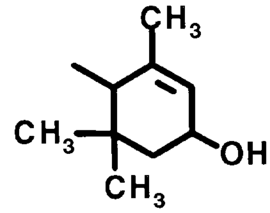

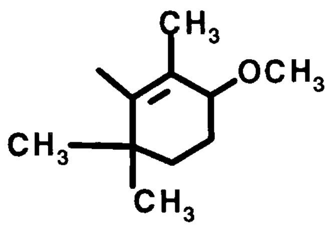
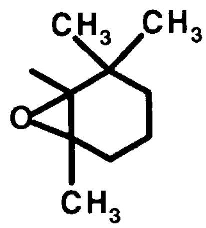
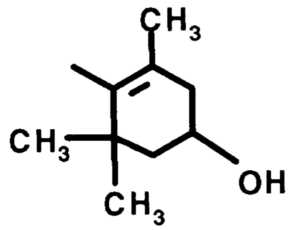

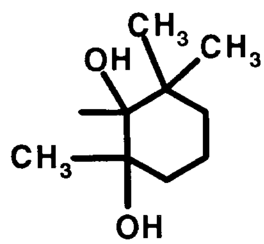
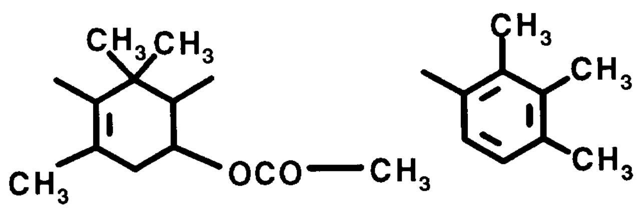

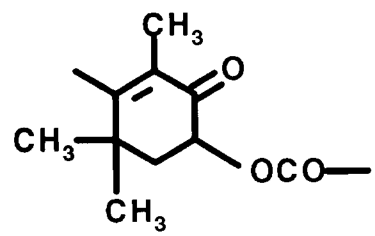 oder 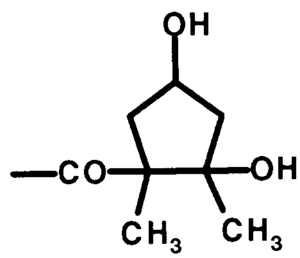

steht, bzw. einer Kombination solcher Wirkstoffe, sowie
0 bis 40 Gewichts-% eines als Hydrotrop, bzw. Co-Emulgator dienenden, pharmaverträglichen Lösungsmittels oder Lösungsmittelgemisches
0,001 bis 90 Gewichts-% eines pharmaverträglichen Tensides oder Tensidgemisches
0 bis 10 Gewichts-% eines Vitamins oder Provitamins
0 bis 10 Gewichts-% pharmaübliche Trägerstoffe und/oder Verdünnungsmittel enthält.

**2.** Spontan dispergierbares Konzentrat gemäss Anspruch 1, dadurch gekennzeichnet, dass es als Tensid ein nicht-ionogenes Tensid mit einem HLB-Wert zwischen 2 und 18, vorzugsweise zwischen 2 und 6 und/oder ein Phosphorsäureester-Tensid enthält.

**3.** Spontan dispergierbares Konzentrat gemäss Anspruch 2, dadurch gekennzeichnet, dass es
7,5 bis 15 Gewichts-% eines oder mehrerer Ester mit Vitamin-E-Verbindungen der Formeln (I) und (II)
0 bis 40 Gewichts-% Isopropylmyristat, Isopropylpalmitat oder Neutralöl
20 bis 45 Gewichts-% Nonylphenol-10-polyoxyäthylen-mono/dimethyl Phosphorsäureester und
20 bis 45 Gewichts-% des wasserfreien tert. Octylphenylpolyoxyäthylenäthers mit 9 bis 10 Oxyäthylengruppen enthält.

4. Spontan dispergierbares Konzentrat gemäss Anspruch 2, dadurch gekennzeichnet, dass es
7,5 bis 15 Gewichts-% eines oder mehrerer Ester mit Vitamin-E-Verbindungen der Formeln (I) und (II)
0 bis 40 Gewichts-% Isopropylmyristat, Isopropylpalmitat oder Neutralöl
20 bis 45 Gewichts-% Tristyrylphenolpolyoxyäthylen-18-mono/dimethyl-Phosphorsäureester und
20 bis 45 Gewichts-% des wasserfreien tert. Octylphenylpolyoxyäthylenäthers mit 9 bis 10 Oxyäthylengruppen enthält.

5. Ester mit Vitamin-E-Verbindungen der allgemeinen Formeln (I) und (II), zur Verwendung als Arzneimittel mit antitumoraler Wirksamkeit :

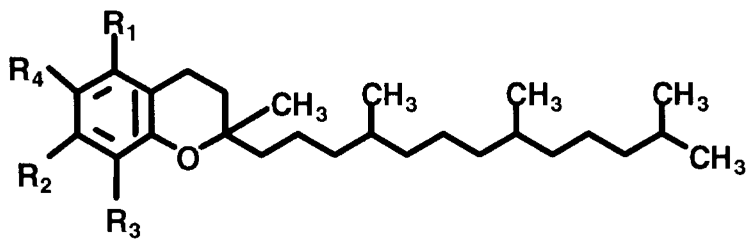

(I)

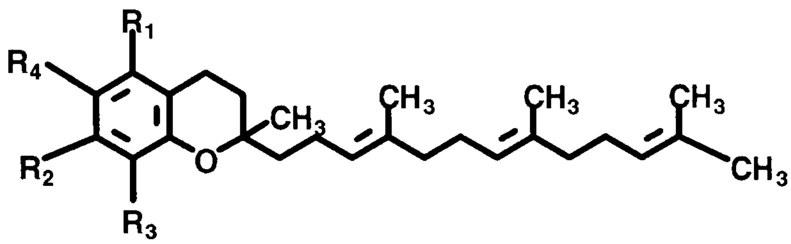

(II)

wobei die Radikale $R_1$, $R_2$ und $R_3$ Wasserstoff oder Methyl bedeuten und das Radikal $R_4$ eine $C_1$- bis $C_{32}$-Alkyl, bzw. eine $C_2$- bis $C_{32}$-Alkenyl- oder Alkapolyenkette mit funktioneller Carboxylgruppe oder eine Gruppe der Formeln (VII) oder (VIII) :

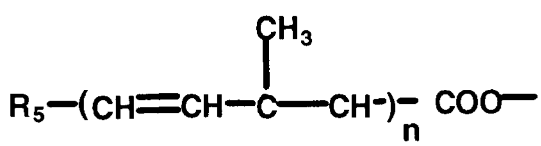

(VII)

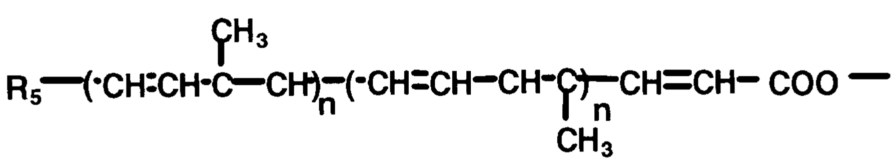

(VIII)

darstellt, worin n die Zahlen 1, 2, 3, 4 oder 5 bezeichnet und $R_5$ für ein Radikal der Formeln

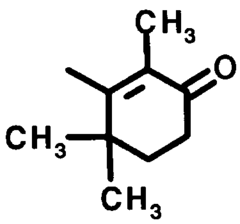
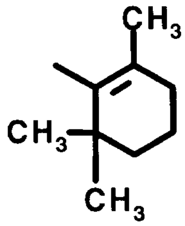
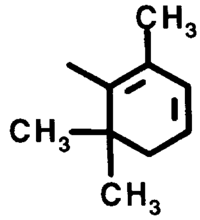
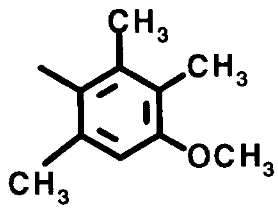
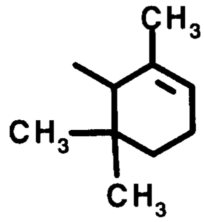
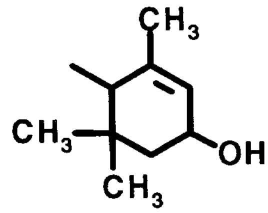
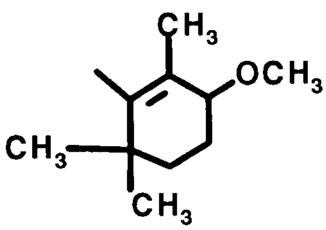
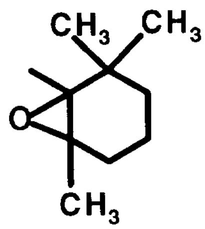
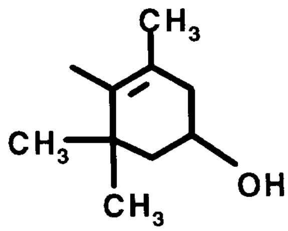
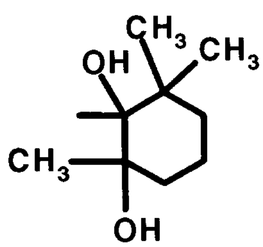
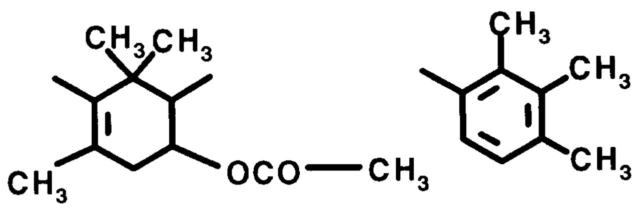

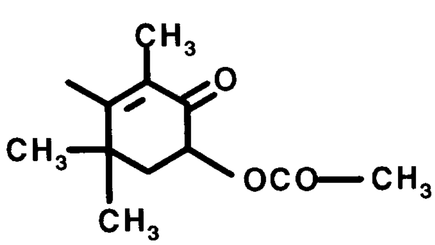 oder 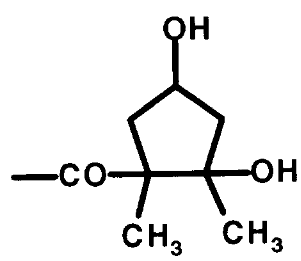

steht.

6. Ester mit Vitamin-D-Verbindungen gemäss Anspruch 5, dadurch gekennzeichnet, dass das Radikal $R_4$ in den Formeln (I) bis (II) eine $C_4$- bis $C_{22}$-Alkylcarboxyl-, bzw. eine $C_4$- bis $C_{22}$-Alkenylcarboxyl- oder eine $C_4$- bis $C_{22}$-Alkapolyencarboxylgruppe bezeichnet oder für ein Radikal der Formeln (VII), (VIII) oder (IX) :

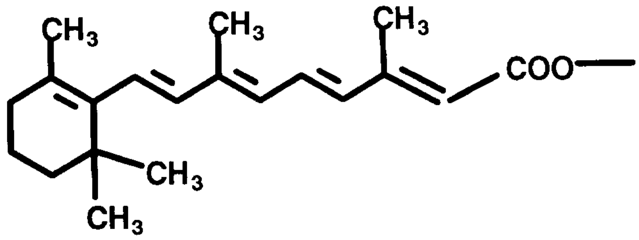

(VII)

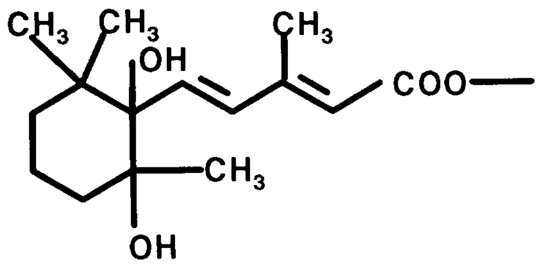

(VIII)

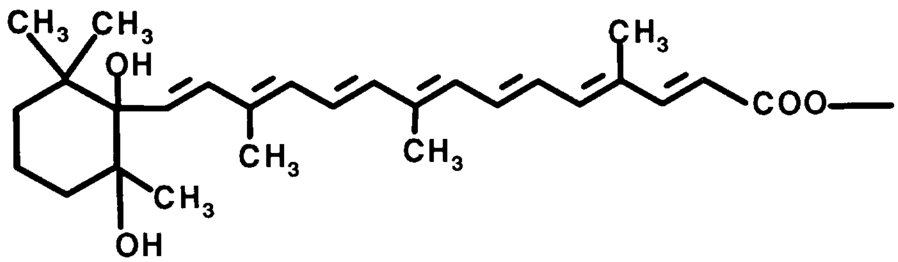

(IX)

steht.

7. Therapeutisches Systempräparat, welches 1 bis 95 Gewichts-% des spontan dispergierbaren Konzentrates gemäss einem der Ansprüche 1 oder 4 enthält und welches in Dosis-Einheitsform als Micropellets, Granulat, Dragées, Suppositorien, Ampullen oder als Kapseln vorliegt.

8. Therapeutisches Systempräparat gemäss Anspruch 7, welches 44 Teile Kernmaterial für die Granulat-, bzw. Pellet-Bildung, 25 Teile eines erfindungsgemässen Konzentrates und 31 Teile magensaftresistente und retardierende Beschichtung mit Hydroxylpropyl-Methylcellulose-Acetat-Succinat enthält.

9. Therapeutisches Systempräparat gemäss Anspruch 7, welches 64 Teile Kernmaterial für die Granulat-, bzw. Pellet-Bildung und 36 Teile eines erfindungsgemässen Konzentrates enthält und in pharmazeutisch geeignete Kapseln aus Hydroxylpropyl-Methylcellulose-Acetat-Succinat abgefüllt wird.

## BATTELLE INSTITUT, FRANKFURT
### IN-VITRO PRÜFUNGEN AUF ZYTOTOXIZITÄT ($LC_{50}$)
### MIT ZELL-LINIEN VON HUMANEN SOLIDEN TUMOREN
### HERBST 1991

EJ 28 BLASENKARZINOM
72 h EXPOSITION, 3 Tage Kultur

FIGUR 1

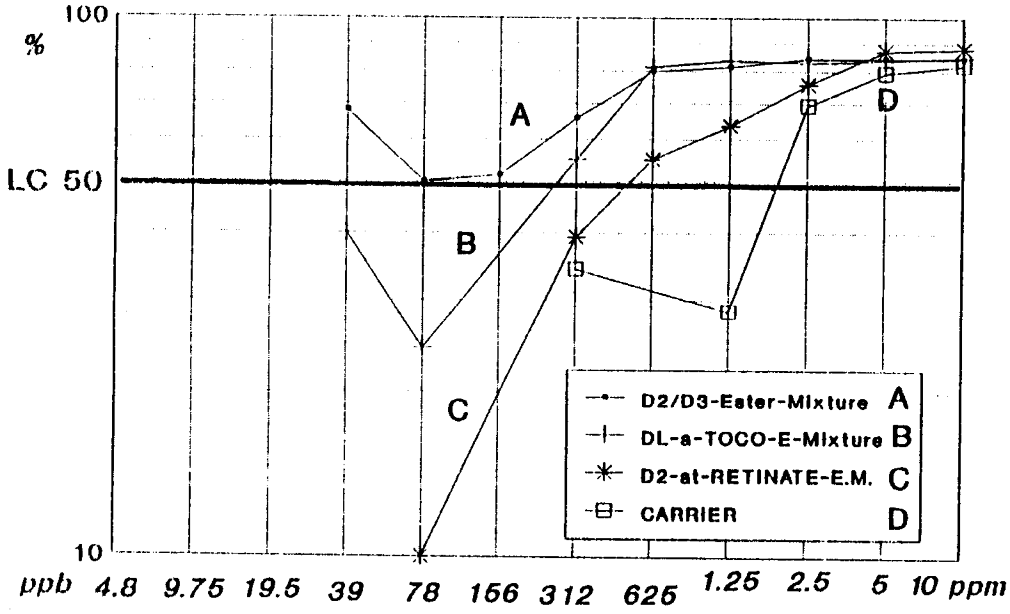


LX-1 LUNGENKARZINOM
72 h EXPOSITION, 3 Tage Kultur

FIGUR 2

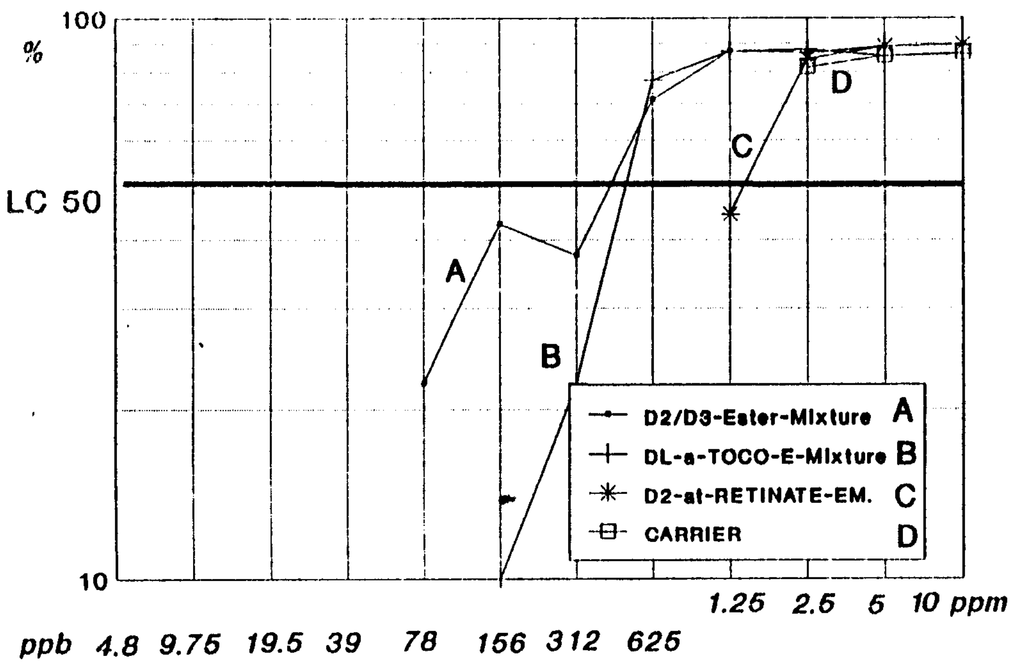

Europäisches Patentamt

# EUROPÄISCHER RECHERCHENBERICHT

Nummer der Anmeldung
EP 94 11 2856

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | EP-A-0 008 573 (NISSHIN FLOUR MILLING)<br>* das ganze Dokument *<br>--- | 5,6 | C07D311/72<br>A61K31/355<br>A61K9/107 |
| X | LIPIDS,<br>Bd.10, Nr.10, Oktober 1975 Champiagn, Ill, US,<br>Seiten 627 - 633<br>T. NAKAMURA, ET AL.: 'Studies on tocopherol derivatives: V. Intestinal absorption if several d,1-3,4-3H2-alpha-tocopheryl esters in the rat'<br>* Tabellen I,II,III *<br>--- | 5,6 | |
| X | DE-A-25 32 000 (EISAI)<br>* Seite 2 *<br>--- | 5,6 | |
| X | GB-A-536 602 (HOFFMANN-LA ROCHE)<br>* das ganze Dokument *<br>--- | 5,6 | |
| X | EP-A-0 231 777 (HOFFMANN-LA ROCHE)<br>* Seite 2 *<br>--- | 5,6 | **RECHERCHIERTE SACHGEBIETE (Int.Cl.5)** |
| X | HELVETICA CHIMICA ACTA,<br>Bd.22, Nr.1, 1939 Basel, CH,<br>Seiten 65 - 67<br>V. DEMOLE, ET AL.: 'Über Ester des alpha-Tocopherols'<br>* Tabelle *<br>--- | 5,6 | C07D<br>A61K |
| X | CHEMICAL ABSTRACTS, vol. 108, no. 15, 11. April 1988, Columbus, Ohio, US;<br>abstract no. 132119d,<br>V.E. ASTROVA,et al.: 'Synthesis of ethers of dl-alpha-tocopherol with higher fatty acids'<br>Seite 786-787 ;<br>& KHIM.-FARM. ZH., 1987, 21(2), 210-213<br>* Zusammenfassung *<br>---<br>-/-- | 5,6 | |

Der vorliegende Recherchenbericht wurde für alle Patentansprüche erstellt

| Recherchenort | Abschlußdatum der Recherche | Prüfer |
|---|---|---|
| DEN HAAG | 7. November 1994 | English, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 94 11 2856

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | CHEMICAL ABSTRACTS, vol. 85, no. 17, 25. Oktober 1976, Columbus, Ohio, US; abstract no. 124243f, Seite 672 ; & JP-A-5 111 113 (BOSO OIL AND FAT) 8. April 1976<br>----- | 5,6 | |
| | | | RECHERCHIERTE SACHGEBIETE (Int.Cl.5) |

Der vorliegende Recherchenbericht wurde für alle Patentansprüche erstellt

| Recherchenort | Abschlußdatum der Recherche | Prüfer |
|---|---|---|
| DEN HAAG | 7. November 1994 | English, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument